(19) Europäisches Patentamt
European Patent Office
Office européen des brevets

(11) EP 0 868 905 A2

(12) # DEMANDE DE BREVET EUROPEEN

(43) Date de publication:
**07.10.1998 Bulletin 1998/41**

(51) Int Cl.[6]: **A61K 7/42**

(21) Numéro de dépôt: **98400493.7**

(22) Date de dépôt: **02.03.1998**

(84) Etats contractants désignés:
**AT BE CH DE DK ES FI FR GB GR IE IT LI LU MC NL PT SE**
Etats d'extension désignés:
**AL LT LV MK RO SI**

(30) Priorité: **07.03.1997 FR 9702759**

(71) Demandeur: **L'OREAL**
**75008 Paris (FR)**

(72) Inventeurs:
- **Forestier, Serge**
  **77410 Claye Souilly (FR)**
- **Richard, Hervé**
  **93420 Villepinte (FR)**
- **Allard, Delphine**
  **92700 Colombes (FR)**
- **Candau, Didier**
  **91570 Bievres (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(54) **Procédé pour améliorer la photostabilité d'un dérivé de dibenzoylméthane par un silane benzalmalonate, procédé de préparation de silanes benzalmalonates**

(57) L'invention se rapporte à un nouveau procédé pour améliorer la stabilité d'au moins un dérivé du dibenzoylméthane vis-à-vis du rayonnement UV consistant à associer audit dérivé de dibenzoylméthane une quantité efficace d'un silane benzalmalonate particulier. L'invention se rapporte également à deux nouveaux procédés de préparation de certains de ces silanes benzalmalonates particuliers.

EP 0 868 905 A2

**Description**

La présente invention se rapporte à un nouveau procédé pour améliorer la stabilité d'au moins un dérivé du dibenzoylméthane vis-à-vis du rayonnement UV consistant à associer audit dérivé de dibenzoylméthane une quantité efficace d'un silane benzalmalonate particulier.

Elle se rapporte également à deux nouveaux procédés de préparation de certains de ces silanes particuliers.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

A cet égard, une famille de filtres UV-A particulièrement intéressante est actuellement constituée par les dérivés du dibenzoylméthane, et notamment le 4-(ter.-butyl) 4'-méthoxy dibenzoylméthane, qui présentent en effet un fort pouvoir d'absorption intrinsèque. Ces dérivés du dibenzoylméthane, qui sont maintenant des produits bien connus en soi à titre de filtres actifs dans l'UV-A, sont notamment décrits dans les demandes de brevets français FR-A- 2326405 et FR-A- 2440933, ainsi que dans la demande de brevet européen EP-A-0114607 ; le 4-(ter.-butyl) 4'-méthoxy dibenzoylméthane est par ailleurs actuellement proposé à la vente sous la dénomination commerciale de "PARSOL 1789" par la Société GIVAUDAN.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que, de façon contraignante, des applications répétées à intervalles de temps réguliers et rapprochés doivent être effectuées par l'utilisateur pour obtenir une protection efficace de la peau contre les rayons UV.

La photostabilisation des dérivés du dibenzoylméthane vis-à-vis du rayonnement UV constitue, à ce jour, un problème qui n'a pas encore été résolu de manière complètement satisfaisante.

Ainsi, il a été proposé dans le document EP-A-0 709 080 d'associer aux dérivés du dibenzoylméthane des dérivés bien spécifiques de benzalmalonate, à savoir des siloxanes benzalmalonates afin de diminuer la photoinstabilité desdits dérivés de dibenzoylméthane. Il est notamment indiqué dans ce document que la structure siloxanique de ces dérivés est essentielle pour obtenir une photostabilisation satisfaisante (voir page 10, ligne 22 de EP-A-0 709 080). Toutefois, ces siloxanes benzalmalonates sont des molécules encombrantes et qui sont complexes à synthétiser.

Or, la Demanderesse vient maintenant de découvrir, contrairement à ce qui était suggéré dans l'état de la technique, qu'en associant aux dérivés du dibenzoylméthane mentionnés ci-dessus une quantité efficace non pas d'un siloxane benzalmalonate, mais d'un silane benzalmalonate, il était possible d'améliorer de manière substantielle et remarquable, la stabilité photochimique (ou photostabilité) de ces mêmes dérivés du dibenzoylméthane.

La Demanderesse a également découvert deux nouveaux procédés de préparation de certains de ces silanes benzalmalonates, ces procédés présentant l'avantage d'être beaucoup plus faciles à mettre en oeuvre que le procédé d'hydrosilylation décrit par exemple dans le document JP 07/330 779.

Ainsi, conformément à un premier objet de la présente invention, il est proposé un nouveau procédé pour améliorer la stabilité d'au moins un dérivé du dibenzoylméthane vis-à-vis du rayonnement UV, consistant à associer audit dérivé du dibenzoylméthane une quantité efficace d'au moins un silane benzalmalonate de formule (I) suivante :

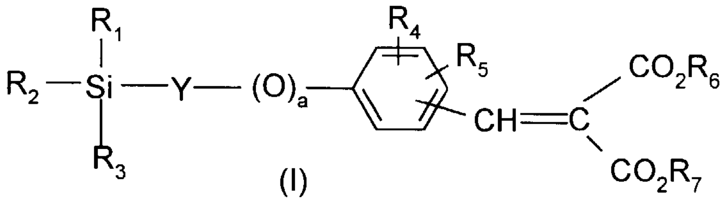

(I)

dans laquelle :

- $R_1$, $R_2$ et $R_3$, identiques ou différents, représentent un radical alkyle en $C_1$-$C_{10}$ éventuellement halogéné ou un radical phényle,
- $R_4$ et $R_5$, identiques ou différents, représentent un atome d'hydrogène, un radical hydroxyle, un radical alkyle en $C_1$-$C_6$, un radical alcoxy en $C_1$-$C_6$ ou un radical triméthylsilyloxy,
- $R_6$ et $R_7$, identiques ou différents, représentent un radical alkyle en $C_1$-$C_8$,
- a est égal à 0 ou 1,
- Y représente un radical divalent répondant à l'une des formules (1) à (4) suivantes :

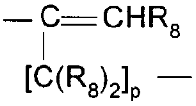

(1)

$$—CR_8 = CH—[C(R_8)_2]_p — \quad (2)$$

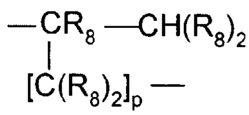

(3)

$$—C(R_8)_2 —CHR_8 —[C(R_8)_2]_p — \quad (4)$$

dans lesquelles :

- $R_8$ représente un atome d'hydrogène ou un radical alkyle en $C_1$-$C_5$,
- p est un nombre entier compris entre 1 et 10 inclus,

étant entendu que le groupe -Y-$(O)_a$- et les deux groupes $R_4$ et $R_5$ sont liés au cycle aromatique indifféremment dans la position para et dans les deux positions méta par rapport au groupe - CH = C - [$(CO_2R_6)$]$(CO_2R_7)$.

Les compositions cosmétiques et/ou dermatologiques obtenues conformément au procédé de l'invention présentent l'avantage d'être particulièrement photostables, même après une exposition prolongée aux rayonnements UV-A et UV-B. Ces rayonnements peuvent être d'origine naturelle (soleil) ou artificielle (lampe UV). De plus, comme décrit ci-après, les silanes benzalmalonates étant des molécules simples à synthétiser, les compositions obtenues selon le procédé conforme à l'invention sont également facilement réalisables industriellement.

La présente invention a également pour objet l'utilisation d'un silane benzalmalonate pour améliorer la stabilité vis-à-vis des rayons UV d'un dérivé du dibenzoylméthane contenu dans une composition cosmétique et/ou dermatologique filtrante.

D'autres caractéristiques, aspects et avantages de la présente invention apparaîtront à la lecture de la description détaillée qui va suivre.

Comme indiqué précédemment, les dérivés du dibenzoylméthane destinés à être photostabilisés selon la présente invention sont des produits déjà bien connus en soi et décrits notamment dans les documents FR-A-2326405, FR-A-2440933 et EP-A-0114607 précités.

Selon la présente invention, on peut bien entendu mettre en oeuvre un ou plusieurs dérivés du dibenzoylméthane.

Parmi les dérivés du dibenzoylméthane utilisables selon la présente invention, on peut notamment citer, de manière non limitative :

- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane,
- le 4,4'-diméthoxydibenzoylméthane.

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société GIVAUDAN, ce filtre répondant donc à la formule développée suivante :

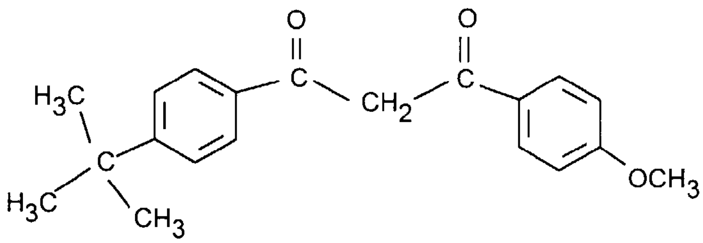

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

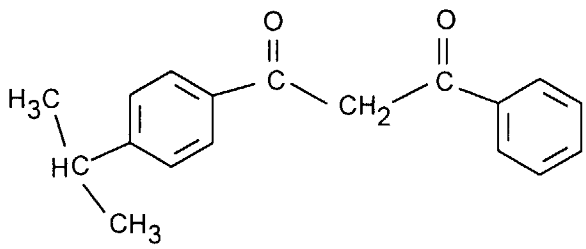

Le ou les dérivés du dibenzoylméthane peuvent être présents dans les compositions destinées à être stabilisées conformément au procédé de l'invention, à des teneurs allant généralement de 0,01 % à 10 % en poids, et de de préférence à des teneurs allant de 0,3 % à 5 % en poids, par rapport au poids total de la composition.

Comme indiqué précédemment, les silanes benzalmalonates utilisables selon la présente invention sont des composés connus qui sont notamment décrits dans le document JP 07/330 779. Ils répondent à la formule (I) suivante :

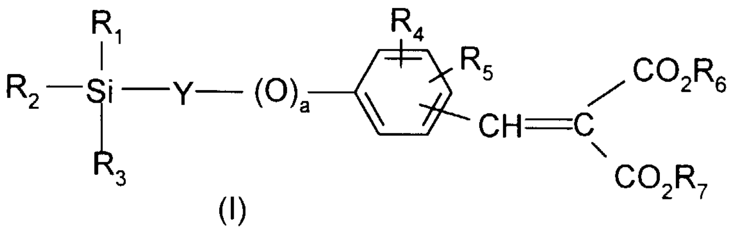

(I)

dans laquelle :

- $R_1$, $R_2$ et $R_3$, identiques ou différents, représentent un radical alkyle en $C_1$-$C_{10}$ linéaire ou ramifié éventuellement halogéné ou un radical phényle,
- $R_4$ et $R_5$, identiques ou différents, représentent un atome d'hydrogène, un radical hydroxyle, un radical alkyle en $C_1$-$C_6$ un radical alcoxy en $C_1$-$C_6$ ou un radical triméthylsilyloxy,
- $R_8$ et $R_7$, identiques ou différents, représentent un radical alkyle en $C_1$-$C_8$,
- a est égal à 0 ou 1,
- Y représente un radical divalent répondant à l'une des formules (1) à (4) suivantes :

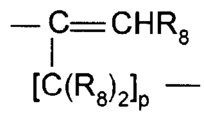

(1)

$$—CR_8 = CH—[C(R_8)_2]_p — \qquad (2)$$

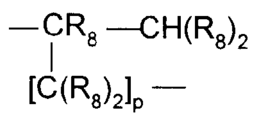

(3)

$$—C(R_8)_2 —CHR_8 — [C(R_8)_2]_p — \qquad (4)$$

dans lesquelles :

- $R_8$ représente un atome d'hydrogène ou un radical alkyle en $C_1$-$C_5$,
- p est un nombre entier compris entre 1 et 10 inclus,

étant entendu que le groupe -Y-$(O)_a$- et les deux groupes $R_4$ et $R_5$ sont liés au cycle aromatique indifféremment dans la position para et dans les deux positions méta par rapport au groupe -CH = C-[$(CO_2R_6)$]$(CO_2R_7)$.

Les silanes benzalmalonates selon l'invention, comme on va le voir plus loin, présentent l'avantage d'être beaucoup plus simples à fabriquer et à purifier que les siloxanes benzalmalonates connus de l'état de la technique. Il en résulte que le procédé de l'invention est également plus facile à mettre en oeuvre à une échelle industrielle par exemple.

Dans une forme préférée de réalisation de l'invention, on met en oeuvre les silanes de formule (I) ci-dessus et répondant au moins à l'une, de préférence à l'ensemble, des caractéristiques suivantes :

- $R_1$ est méthyle,
- $R_2$ est méthyle ou éthyle,
- $R_3$ est méthyle,
- Y représente un radical divalent de formule (3) ou (4) dans lesquelles $R_8$ est l'hydrogène ou méthyle et p est 1 ou 2,
- a est 1,
- $R_4$ est l'hydrogène ou un groupe méthoxy,
- $R_5$ est l'hydrogène,
- $R_6$ est méthyle ou éthyle

- $R_7$ est méthyle ou éthyle
- le groupe $-Y-(O)_a-$ est lié au cycle aromatique dans la position para par rapport au groupe $-CH=C-[(CO_2R_6)](CO_2R_7)$.

Des silanes convenant particulièrement bien à la réalisation de la présente invention sont les composés répondant aux formules (5), (6) et (7) suivantes :

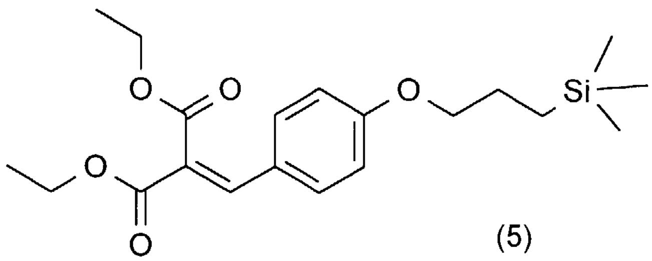

(5)

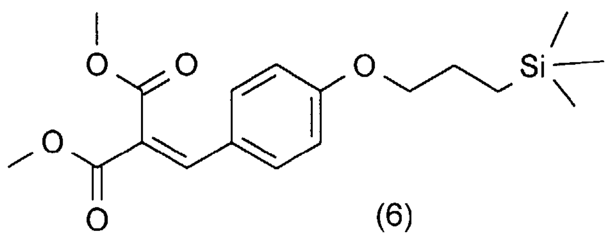

(6)

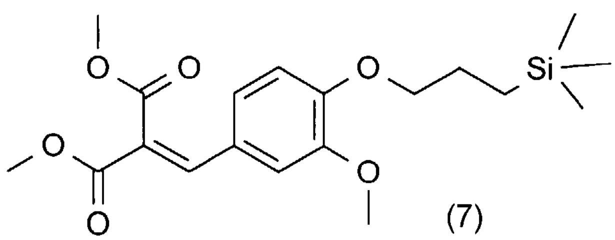

(7)

Pour préparer les dérivés de formule (I) dans lequel a = 0, on peut faire une réaction d'hydrosilylation entre le dérivé insaturé correspondant et le silane correspondant tel qu'il est décrit dans le brevet JP 07/330 779.

La présente invention a encore pour objet un premier procédé de préparation (voie A) des dérivés de formule (I) dans laquelle a est 1 consistant dans un premier temps à faire réagir un hydroxybenzaldéhyde aromatique de formule (8) ci-dessous sur un dérivé silanique halogéné de formule (9) ci-dessous, puis dans un deuxième temps à condenser le benzaldéhyde de formule (10) ainsi obtenu avec un diester malonique de formule (11) ci-dessous. De préférence, on fait réagir l'hydroxy benzaldéhyde aromatique et le dérivé silanique halogéné en présence d'une base selon une réaction d'alkylation classique, la condensation du benzaldéhyde obtenu par le diester malonique se faisant dans le toluène en présence d'acétate de piperidinium comme catalyseur (condensation de Knoevenagel) selon le schéma réactionnel suivant :

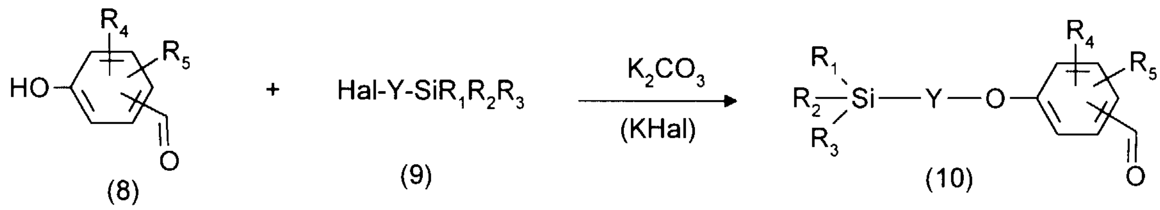


(8) (9) (10)

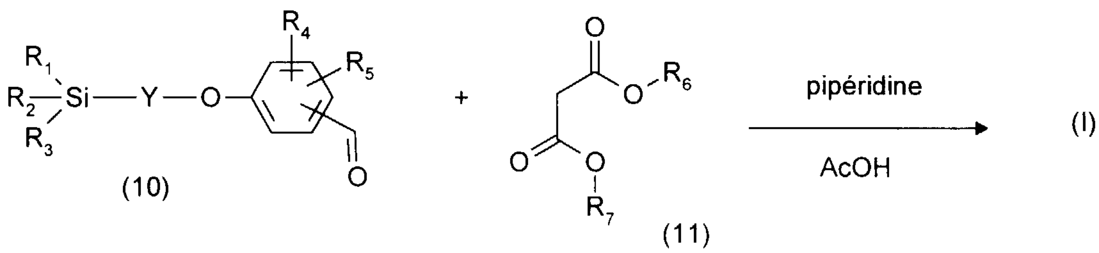


(10) (11)

dans lequel Y, $R_1$, $R_2$, $R_3$, $R_4$, $R_5$, $R_6$ et $R_7$ ont les significations indiquées ci-dessus pour la formule (I) et Hal représente un halogène, de préférence le chlore.

La présente invention a également pour objet un deuxième procédé de préparation (voie B) des dérivés de formule (I) dans laquelle a est 1 consistant à faire réagir un hydroxybenzalmalonate de formule (12) ci-dessous sur un dérivé silanique halogéné de formule (9). De préférence, on fait réagir l'hydroxybenzalmalonate et le dérivé silanique halogéné en présence d'une base selon une réaction d'alkylation classique selon le schéma suivant :

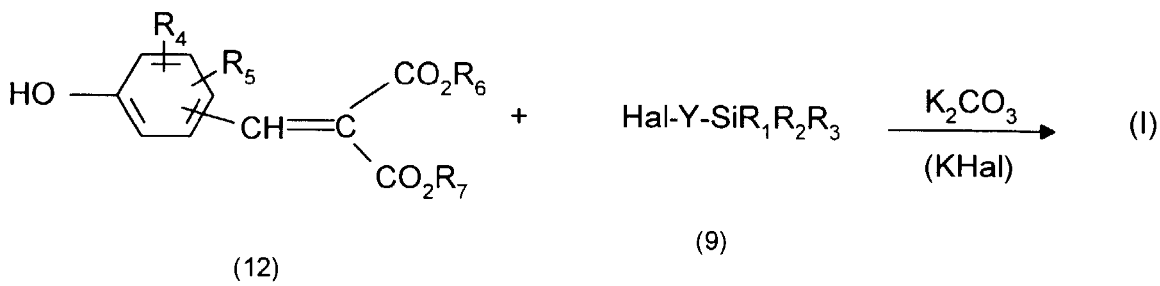


(12) (9)

dans lequel Y, $R_1$, $R_2$, $R_3$, $R_4$, $R_5$, $R_6$, et $R_7$ ont les mêmes significations qu'à la formule (I) ci-dessus et Hal représente un halogène, de préférence le chlore.

Ces méthodes de synthèse présentent l'avantage d'être plus faciles à réaliser que les réactions d'hydrosilylation.

Comme dérivés de benzaldéhyde aromatiques, on peut par exemple citer l'hydroxy-4 benzaldéhyde ou la vanilline qui sont des produits commerciaux.

Comme dérivés d'ester malonique, on peut par exemple citer le malonate de diméthyle ou le malonate diéthyle qui sont des produits commerciaux.

Comme dérivé d'hydroxybenzalmalonates, on peut par exemple citer le para-hydroxy benzalmalonate de diméthyle vendu par la société ACROS.

Comme dérivés d'halogénure silanique, on peut par exemple citer le chloropropyl triméthyl silane vendu par la société WACKER.

Par quantité efficace de silane benzalmalonate selon l'invention, on entend une quantité suffisante pour obtenir une amélioration notable et significative de la photostabilité du ou des dérivés du dibenzoylméthane contenus dans la composition. La quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que décrit dans la demande FR-A-2607700.

Les silanes benzalmalonates sont généralement présents dans les compositions selon l'invention à une teneur au moins égale à 0,5 % en poids, par rapport au poids total de la composition. De préférence encore, cette teneur va de 0,5 % à 20 % en poids, par rapport au poids total de la composition.

Les compositions cosmétiques et/ou dermatologiques visées par la présente invention peuvent bien entendu contenir un ou plusieurs filtres solaires complémentaires actifs dans l'UVA et/ou l'UVB (absorbeurs), hydrophiles ou lipophiles. Ces filtres complémentaires peuvent être notamment choisis parmi les dérivés cinnamiques, les dérivés salicyliques, les dérivés du benzylidène camphre, les dérivés de benzimidazole, les dérivés de triazine, les dérivés de la benzophénone, les dérivés de β,β'-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A-487404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-518772 et EP-A-518773.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (comme le benzoate d'alcools en $C_{12}$-$C_{15}$ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus (en particulier les filtres complémentaires) et/ou leurs quantités de manière telle que les propriétés avantageuses attachées intrinsèquement au procédé conforme à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique et/ou dermatologique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour

cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation.

Des exemples concrets, mais nullement limitatifs, illustrant l'invention, vont maintenant être donnés.

**EXEMPLE 1** :

Préparation du 2-[3-méthoxy-4-(3-triméthylsilanyl-propyloxy)-benzylidène]-malonic acid diméthyl ester (selon la Voie A)

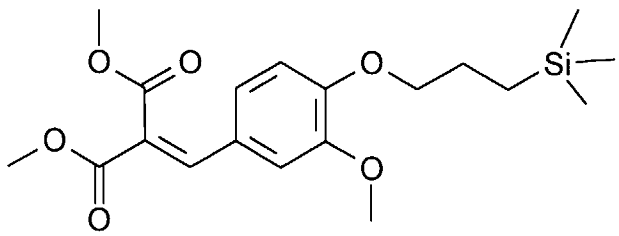

a) Première étape : préparation du 3-méthoxy-4-(3-triméthylsilanyl-propyloxy)-benzaldéhyde :

Dans un mélange de vanilline (30,4 g, 0,2 mole) et de carbonate de potassium (30,4 g, 0,22 mole) dans 150 ml de diméthylformamide (DMF) sec porté à 80°C sous azote, on ajoute goutte à goutte en 20 minutes du 3-chloropropyltriméthylsilane (33,14 g, 0,22 mole). On laisse 4 heures à 95-110°C. On refroidit et verse le mélange réactionnel dans de l'eau glacée. La phase aqueuse est extraite 3 fois au dichlorométhane. Les phases organiques sont séchées sur sulfate de sodium et concentrées sous vide. Après distillation sous vide (0,04 mmHg), on obtient 47,5g (Rendement : 89 %) de 3-méthoxy-4-(3-triméthylsilanyl-propyloxy)-benzaldéhyde sous forme d'une huile légèrement rosée distillant à 112-114°C et utilisée telle quelle dans l'étape suivante.

b) Deuxième étape : préparation du 2-[3-méthoxv-4-(3-triméthylsilanyl-propyloxy)-benzylidène]-malonic acid diméthyl ester:

On porte au reflux pendant 3 heures un mélange du dérivé précédent (13,3 g, 0,05 mole) et de malonate de diméthyle (7,93 g, 0,06 mole) dans 20 ml de toluène en présence de 0,5 ml de pipéridine et de 0,3 ml d'acide acétique. On refroidit et on verse le mélange réactionnel dans l'eau puis on extrait à l'éther diisopropylique. La phase organique est séchée sur sulfate de sodium et concentrée. Après cristallisation dans un mélange volumique méthanol/eau 90: 10, on obtient 15 g (Rendement 79 %) du dérivé ayant les caractéristiques suivantes :

- poudre blanche
- Pf : 51-52°C
- UV (Ethanol) $\lambda_{max}$ = 331 nm, $\varepsilon_{max}$ = 20 300

| Analyse élémentaire pour $C_{19} H_{28} O_6 Si$ | | | |
|---|---|---|---|
| théorie : | C : 59.97 | H : 7.42 | Si : 7.38 |

(suite)

| Analyse élémentaire pour $C_{19} H_{28} O_6 Si$ | | | |
|---|---|---|---|
| trouvé : | C : 59.80 | H : 9.35 | Si : 7.30 |

EXEMPLE 2:

Préparation du 2-[4-(3-triméthylsilanyl-propyloxy)-benzylidène]-malonic acid diéthyl ester (selon la Voie B)

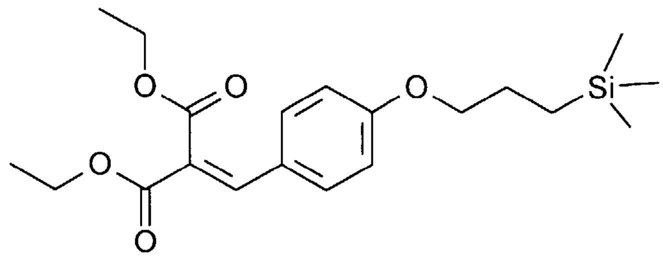

Dans un mélange de 4-hydroxy benzalmalonate de diéthyle (15,18 g, 0,11 mole) et de carbonate de potassium (15,18 g, 0,11 mole) dans 100 ml de DMF sec porté à 80°C sous azote, on ajoute goutte à goutte en 10 minutes du 3-chloropropyltriméthylsilane (16,57 g, 0,11 mole). On laisse 3 heures 30 minutes à 120-130°C. On refroidit et on verse le mélange réactionnel dans de l'eau glacée. La phase aqueuse est extraite 2 fois au dichlorométhane. Les phases organiques sont lavées à l'eau puis séchées sur sulfate de sodium et concentrées sous vide. On obtient après cristallisation dans le méthanol 31,6 g (Rendement : 76 %) du dérivé ayant les caractéristiques suivantes :

- poudre blanche
- Pf : 34-35°C
- UV (Ethanol) $\lambda_{max}$ = 314 nm, $\varepsilon_{max}$ = 26 400

| Analyse élémentaire pour $C_{20} H_{30} O_5 Si$ | | | |
|---|---|---|---|
| théorie : | C : 63.46 | H : 7.99 | Si : 7.42 |
| trouvé : | C : 63.31 | H : 7.90 | Si : 7.50 |

La Demanderesse a constaté que le silane benzalmalonate obtenu ci-dessus permettait de stabiliser le 4-tert.-butyl-4'-méthoxydibenzoylméthane vis-à-vis du rayonnement ultraviolet de façon au moins aussi efficace que les siloxanes benzalmalonates décrits dans le document EP-A-0709080.

**Revendications**

1. Procédé pour améliorer la stabilité d'au moins un dérivé du dibenzoylméthane vis-à-vis du rayonnement UV, caractérisé par le fait qu'il consiste à associer audit dérivé du dibenzoylméthane une quantité efficace d'au moins un silane benzalmalonate de formule (I) suivante :

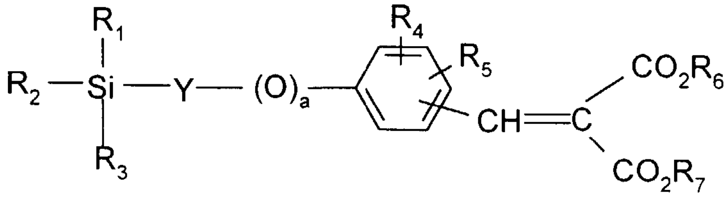

(I)

dans laquelle :

- $R_1$, $R_2$ et $R_3$, identiques ou différents, représentent un radical alkyle en $C_1$-$C_{10}$ éventuellement halogéné ou un radical phényle,
- $R_4$ et $R_5$, identiques ou différents, représentent un atome d'hydrogène, un radical hydroxyle, un radical alkyle en $C_1$-$C_6$ un radical alcoxy en $C_1$-$C_6$ ou un radical triméthylsilyloxy,
- $R_6$ et $R_7$, identiques ou différents, représentent un radical alkyle en $C_1$-$C_8$,
- a est égal à 0 ou 1,
- Y représente un radical divalent répondant à l'une des formules (1) à (4) suivantes :

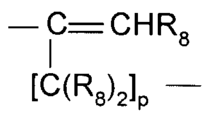

(1)

$$—CR_8 = CH—[C(R_8)_2]_p — \quad (2)$$

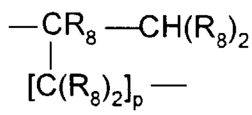

(3)

$$—C(R_8)_2 —CHR_8—[C(R_8)_2]_p — \quad (4)$$

dans lesquelles :

- $R_8$ représente un atome d'hydrogène ou un radical alkyle en $C_1$-$C_5$,
- p est un nombre entier compris entre 1 et 10 inclus,

étant entendu que le groupe -Y-$(O)_a$- et les deux groupes $R_4$ et $R_5$ sont liés au cycle aromatique indifféremment dans la position para et dans les deux positions méta par rapport au groupe - CH = C - [$(CO_2R_6)$]$(CO_2R_7)$.

**2.** Procédé selon la revendication 1, caractérisé en ce que le silane benzalmalonate répond au moins à l'une, de préférence à l'ensemble, des caractéristiques suivantes :

- $R_1$ est méthyle,
- $R_2$ est méthyle ou éthyle,
- $R_3$ est méthyle,
- Y représente un radical divalent de formule (3) ou (4) dans lesquelles $R_8$ est l'hydrogène ou méthyle et p est 1 ou 2,
- a est 1,
- $R_4$ est l'hydrogène ou un groupe méthoxy,
- $R_5$ est l'hydrogène,
- $R_6$ est méthyle ou éthyle
- $R_7$ est méthyle ou éthyle
- le groupe -Y-$(O)_a$- est lié au cycle aromatique dans la position para par rapport au groupe -CH=C-[(CO2R6)] $(CO_2R_7)$.

**3.** Procédé selon la revendication 1 ou 2, caractérisé par le fait que le silane benzalmalonate répond à l'une des formules (5), (6) ou (7) suivantes :

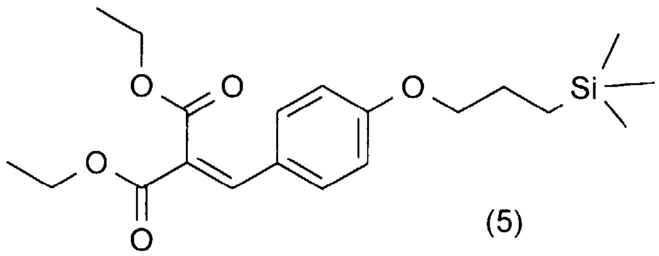

(5)

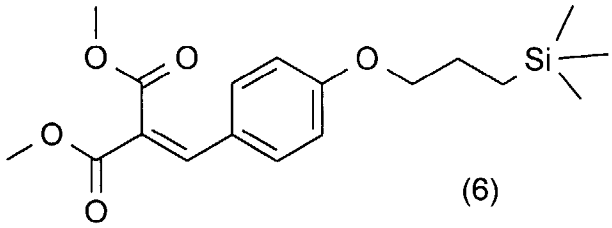

(6)

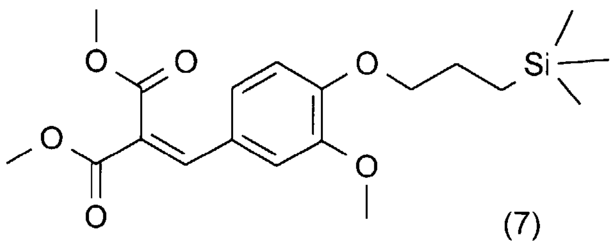

(7)

4. Procédé selon l'une quelconque des revendications précédentes, caractérisé par le fait que le dérivé de dibenzoylméthane est choisi parmi :

- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

5. Procédé selon la revendication 4, caractérisé par le fait que le dérivé de dibenzoylméthane est le 4-(ter.-butyl) 4'-méthoxy dibenzoylméthane.

6. Procédé selon la revendication 4, caractérisé par le fait que le dérivé de dibenzoylméthane est le 4-isopropyl-dibenzoylméthane.

7. Utilisation d'un silane benzalmalonate de formule (I) tel que défini à l'une quelconque des revendications 1 à 3 pour améliorer la stabilité d'au moins un dérivé du dibenzoylméthane vis-à-vis du rayonnement UV.

**8.** Procédé de préparation des composés de formule (I) tels que définis à la revendication 1 pour lesquels a est 1, caractérisé par le fait qu'il consiste dans un premier temps à faire réagir un hydroxybenzaldéhyde aromatique de formule (8) suivante :

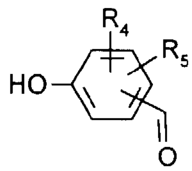

(8)

sur un dérivé silanique halogéné de formule (9) suivante :

$$Hal\text{-}Y\text{-}SiR_1R_2R_3 \qquad (9)$$

puis dans un deuxième temps à condenser le benzaldéhyde ainsi obtenu avec un diester malonique de formule (11) suivante :

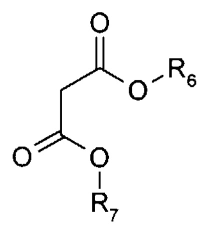

(11)

formules (8), (9) et (11) dans lesquelles Y, $R_1$, $R_2$, $R_3$, $R_4$, $R_5$, $R_6$, et $R_7$ ont les mêmes significations qu'à la formule (I) de la revendication 1 et Hal représente un halogène.

**9.** Procédé selon la revendication 8, caractérisé par le fait qu'il est mis en oeuvre selon le schéma réactionnel suivant :

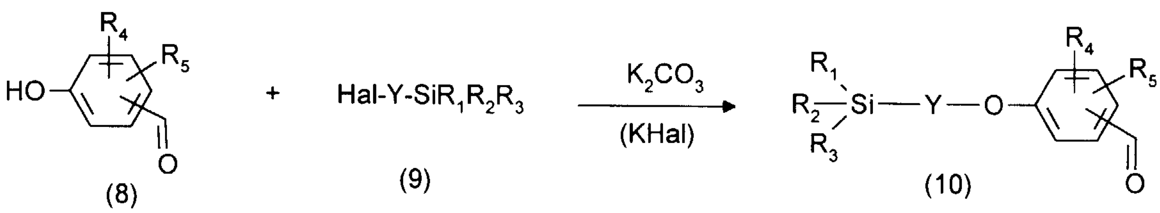


(8) (9) (10)

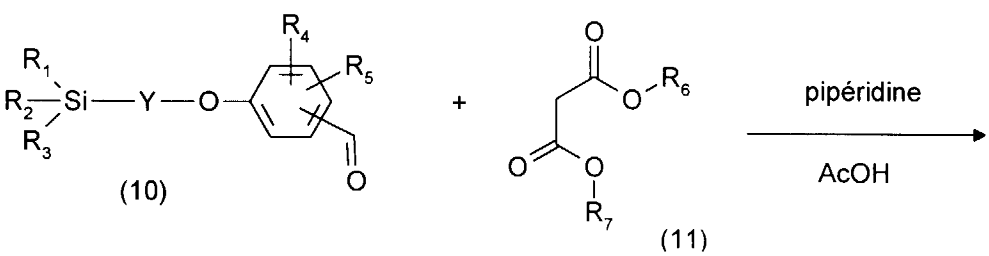


(10) (11)

dans lequel Y, $R_1$, $R_2$, $R_3$, $R_4$, $R_5$, $R_6$, et $R_7$ ont les mêmes significations qu'à la formule (I) de la revendication 1 et Hal représente un halogène, de préférence le chlore.

**10.** Procédé de préparation des composés de formule (I) tels que définis à la revendication 1 pour lesquels a est 1, caractérisé par le fait qu'il consiste à faire réagir un hydroxybenzalmalonate de formule (12) suivante :

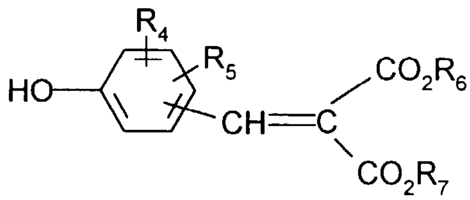

(12)

sur un dérivé silanique halogéné de formule (9) suivante :

$$Hal\text{-}Y\text{-}SiR_1R_2R_3 \qquad (9)$$

formules (9) et (12) dans lesquelles Y, $R_1$, $R_2$, $R_3$, $R_4$, $R_5$, $R_6$, et $R_7$ ont les mêmes significations qu'à la formule (I) de la revendication 1 et Hal représente un halogène.

**11.** Procédé selon la revendication 10, caractérisé par le fait qu'il est mis en oeuvre selon le schéma réactionnel suivant :

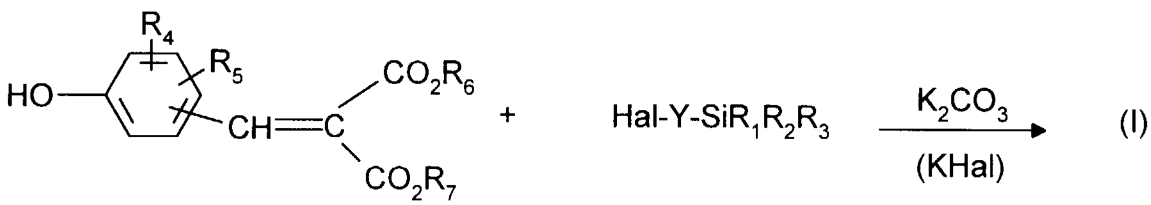


(12) (9)

dans lequel Y, $R_1$, $R_2$, $R_3$, $R_4$, $R_5$, $R_6$, et $R_7$ ont les mêmes significations qu'à la formule (I) de la revendication 1 et Hal représente un halogène, de préférence le chlore.